# EUROPEAN PATENT SPECIFICATION

(11) **EP 3 372 669 B1**
(45) Date of publication and mention of the grant of the patent: **14.10.2020**
(21) Application number: 15907892.2
(22) Date of filing: 03.11.2015
(51) Int. Cl.: C12M 3/06, C12M 1/12, C12N 5/077, A61K 35/12, A61P 41/00

(54) **DEVICE FOR ISOLATING CELL FRACTIONS FROM HUMAN AND ANIMAL TISSUES AND METHOD FOR THE USE THEREOF**
VORRICHTUNG ZUR ISOLIERUNG VON ZELLFRAKTIONEN AUS MENSCHLICHEM UND TIERISCHEM GEWEBE UND VERFAHREN ZUR VERWENDUNG DAVON
DISPOSITIF D'EXTRACTION DE FRACTIONS CELLULAIRES À PARTIR DE TISSUS HUMAINS ET ANIMAUX ET PROCÉDÉ DE SON UTILISATION

(43) Date of publication of application: 12.09.2018
(73) Proprietor: Jointechcell, Limited Liability Company (JTC LLC), Moscow 125252 (RU)
(72) Inventor: VEREMEYEV, Aleksey Vladimirovich, Moscow 652702 (RU); KATS, Natan Georgiyevich, Shtat Illinoys (US); NESTERENKO, Vladimir Georgiyevich, Moscow 125009 (RU); BOLGARIN, Roman Nikolayevich, Pushchino Moskovskaya obl. 142290 (RU); PETKOVA, Mariya Aleksandrovna, Moscow 107076 (RU)
(74) Representative: Comoglio, Elena
(86) International application number: PCT/RU2015/000738
(87) International publication number: WO 2017/078563

(56) References cited:
- WO-A1-2012/148350
- WO-A1-2013/106655
- WO-A1-2014/039697
- WO-A1-2015/035221
- RU-C1- 2 252 252
- US-A- 5 372 945
- US-A1- 2001 047 966
- SEVERIANO DOS-ANJOS VILABOA ET AL: "Age influence on stromal vascular fraction cell yield obtained from human lipoaspirates", CYTOTHERAPY, vol. 16, no. 8, 1 August 2014 (2014-08-01) , pages 1092-1097, XP055597121, GB ISSN: 1465-3249, DOI: 10.1016/j.jcyt.2014.02.007
- KOPTEVA V. B.: 'Opory vertikalnykh i gorizontalnykh apparatov' METODICHESKIE UKAZANIA 2005, page 1, 2, XP009506403
- YUAN S. ET AL.: 'Evaluation of embryonic age and the effects of different proteases on the isolation and primary culture of chicken intestinal epithelial cells in vitro' ANIMAL SCIENCE JOURNAL vol. C. 1-7, 2014, page 1,4, XP055487364
- GIMBLE J.M. ET AL.: 'Concise Review: Adipose-Derived Stromal Vascular Fraction Cells and Stem Cells: Let's Not Get Lost in Translation' STEM CELLS vol. 29, no. 5, 19 April 2011, pages C749 - 754, XP055382306

## Description

### TECHNICAL FIELD

The present group of inventions relates to medical biotechnology and cell technology and is intended for isolating cell fractions from human and animal tissues, namely isolating stromal vascular fraction of adipose tissue.

### PRIOR ART

The subcutaneous adipose tissue is a source of stem and progenitor cells for regenerative medicine as an alternative to bone marrow. The main advantage of adipose tissue is the small invasiveness of the retrieval procedures, minimal soreness, as well as minimal discomfort and risk in patients. Adipose tissue provides more stem cells compared to bone marrow. Intramedullary transplant contains 0.006 - 0.06x10⁶ stem cells per 100 ml; and the viable stem and progenitor cells count in adipose tissue fraction is about 0.5x106 to 20x10⁶ cells per 100 ml of tissue.

The heterogeneous fraction of adipose tissue, separated from stroma and adipocytes with collagenase, is called stromal vascular fraction. The stromal vascular fraction is characterized by significant heterogeneity and variability of the cell composition depending on donor state as well as age and place of collection of biological material. After sorting, the following cell populations can be isolated from the stromal vascular fraction: endothelial and smooth muscle cells, pericytes, fibroblasts, fat cells, and preadipocytes. The regenerative fraction cells, namely the stem and progenitor cell complex of adipose tissue (adipose derived stem cells, ADSCs), are of interest. ADSCs have significant potential to differentiate into adipocytes, chondrocytes and osteoblasts, myocytes, neuronal cells, cardiomyocytes, and hepatocytes.

The clinical use of the stromal vascular fraction includes soft tissue and bone regeneration, cosmetic defects, chronic trophic and radiation ulcers, burns, Crohn's disease, multiple sclerosis, graft versus host reaction, myocardial infarction, and strokes of various genesis. The main advantages of using this cell type are easy access to the cell source, a large number and high yield per unit volume of tissue, high multipotency, comparable efficacy and safety regarding the bone marrow mesenchymal cells. Besides, the lipoaspiration procedure is widely used; it is standardized, simple in execution in a treatment room, and takes only about 90 minutes. Thus, cell fractions isolated from adipose tissue can be beneficial for the clinical practice by excluding the cultivation step as well.

A method for stem cells isolation from adipose tissue by using a manual method is disclosed (Zuk R. A., Zhu M., Mizuno H. et al. Multilineage cells from human adipose tissue: implications for cell-based therapies. Tissue Eng. 2001; 7(2): 211- 28). The method is based on treatment of lipoaspirate with 0.075% collagenase type I solution at 37°C for 30 minutes and subsequent centrifugation of the resulting suspension at 800 g. After centrifugation, the cell suspension is divided into two fractions: adipocytes in the upper supernatant layer, and in the sediment a stromal vascular fraction with red cells contamination, which were removed during incubation in ammonium chloride lysis solution. The disadvantages and drawbacks of the method are additional time and organization expenses, the "human factor" presence, a high risk of failure of the sterility process, and biological material/end product contamination.

The isolation technologies are developing towards process automation. Automatic and semi-automatic systems of cell isolation have been created.

From the prior art, a device is known (System for processing lipoaspirate cells EP 1921133 A2, CYTORI THERAPEUTICS, INC (US), IPC A61K31/436, published on May 14, 2008), which is a closed system for treating lipoaspirate obtained during liposuction. The system is connected directly to the lipoaspiration cannula. It includes a vacuum pump, a container for collecting the lipoaspirate, a mixer for mixing the processed biomaterial with additives and activators, and a system of two filters with different-diameter pores. The first filter separates the lipoaspirate into 2 fractions, where one fraction contains a cell population including adipose tissue stem cells, and the other fraction contains lipids, blood, adipocytes, and saline. Thereby, the first filter rotates and constructively divides the container into two chambers for the respective fractions, and the second filter concentrates the cell fraction before feeding it into the mixer. The main line allows the end fraction to be retrieved without a breach of the system leakproofness and direct it into centrifuge, then retrieve aseptically the resulting fraction. The device allows the introduction of activators and additives.

The system also has a built-in temperature controller. The main disadvantages and drawbacks of the said device are the following: during the first stage of treatment, the lipoaspirate is filtered without providing tissue stroma destruction which fixes the stromal vascular fraction cells, and it does not allow them to pass through the filter, thereby reducing the cell concentration in the permeate. The system disadvantages and drawbacks include also elongated lines and a significant number of junctions which are the medium for adhesion and loss of target cells.

From the prior art, a device is known (Method and Apparatus for Separating a Material, US 20110251041 A1, BIOMET BIOLOGICS, LLC (US), IPC B04B1/08, published on October 13, 2011), which is a system for multicomponent material separation, at least in part, into two fractions. The said device is a container, in the form of a hollow cylinder divided by a valve into two parts with unequal volumes. The valve function is to distinguish fractions of different densities. At the base of the structure with inclined walls, the system contains a sump separated from the main chamber volume by a valve. The valve has a conical surface; it includes a liner also with conical surface between the side wall and the longitudinal axis of the cylinder; the conical surface is turned with the corner downwards. The valve contains a disc with holes of circular and sector shapes. The valve has a flexible membrane that activates it at increasing hydraulic pressure during centrifugation; when the disk rotates, the holes are juxtaposed, thus ensuring valve permeability. The system is additionally equipped with a chopper and a vacuum pump connected to the separating chamber. In this case, the adipose tissue is separated into three fractions. The first fraction is isolated between the valve and the chamber bottom and contains cellular material. The second fraction is isolated between the valve and the upper part of the chamber and contains fat. The third fraction is isolated between the valve components and contains tumescent fluid. The main disadvantage and drawback of the said system is the danger for the mechanical device elements to be filled with tissue fragments, which entails the risk of device failure and end fraction contamination with original biomaterial components. Treatment without ensuring the destruction of the tissue stroma, which fixes the stromal vascular fraction cells and does not allow them to pass through the filter, thereby reducing the cell concentration in the permeate.

From the prior art, a device is known (Regenerative gel extraction device WO 2013183797 A1, HURIMBIOCELL CO., LTD (KR), IPC C12M1/10, published on December 12, 2013), which is a container with a cone-shaped bottom and a rotating longitudinal axis driven by an external motor. The product composed of a number of petals, which have a direct effect on the lipoaspirate, is fixed on the axis. The reservoir has a nozzle for biomaterial injection and isolating the tissue fraction containing stem cells. The disadvantage and drawback of the said system is a significant mechanical effect on the cells leading to their destruction and damage by released enzymes of other cells, end product contamination with components of the original lipoaspirate, a high risk of biomaterial microbiological contamination due to the absence of aseptic barrier.

From the prior art, a device is known (Device for separating adult stem cell US 20130344589 A1, HUMAN MED AG (DE), IPC C12M1/00, published on December 26, 2013), which is a system including a container for adipose tissue with a liquid feed device for washing the biomaterial, mixtures of the necessary reagents, alternately with the extraction of stem cells fraction, a pressure-balancing valve, a piston, a vibrator, a semi-permeable membrane with electrostatic charge, and a valve dividing the container into 2 parts, as well as a temperature controller. One of the chambers is equipped with a device for mixing the original biomaterial with additives, performing rotational and/or pendulum motions. The main disadvantage and drawback of the said device is the method of pressurization realized by a piston, which leads to a high mechanical stress on the target cells due to the pressing through filter and to marginal cell destruction at the piston junction to the cylinder wall.

As a prototype, a device for isolating adipose tissue stem cells has been chosen (System for methods for the preparation of adipose-derived stem cells, US 20130012921 A1, PUSTILNIK FELIX, IPC A61M37/00, published on January 10, 2013), which is a cone-shaped container, hermetically sealed with a lid provided with connectors for injecting and removing biomaterial and fluids for its treatment, as well as an auxiliary tube for concentration of the resulting stromal vascular fraction. The disadvantages of the said system are the presence of two tube-containers, which increases the risk of material contamination, its loss when transferred between the containers, and entails additional requirements to the peripheral equipment. Besides, there is no separation of the primary lipoaspirate into fractions containing target cells and processing by-products contaminating the end product. Further devices are known from WO2013/106655 A1.

### DISCLOSURE OF THE INVENTION

A common technical task of the said group of inventions is to increase the efficiency and safety of the cell isolation method, to expand its functionality, and optimize the isolation procedure.

The general technical result of the proposed group of inventions is the controlled isolation of adipose tissue stromal vascular fraction characterized by high cell survival, absence of debris, residues of stromal and adipose tissue and circulating blood cells, low concentration and activity of the residual enzymes.

The assigned technical task is achieved by the presence in the device of two chambers separated by a system of microfilters with different pore diameters; the presence of isolated channels in the wall of the system; the presence of valves on the adapter fittings for injection of biological material, addition of reagents and washing buffer, extraction of debris and residues of stromal adipose tissue, extraction of the end product, use of anti-adhesion treatment of the device internal surface, and use a Luer-Lock syringe with an elongated nose and an enlarged piston handle for embedding and extraction of biological material.

The proposed device is a closed, sealed system in the form of transparent cone-shaped 150-300 ml container with external calibration by volume, channel systems and adapters with Luer-Lock type connectors, wherein the container is divided by a multilayer filter into two chambers: a working chamber for tissue treatment and a chamber for cell concentrating, wherein the working chamber has a volume of 145 to 299 ml and the concentration chamber has a volume of 1-5 ml.

The filter system is a complex of at least 2 nylon filters with a 10 µm with diameter of the lower filter and up to 500 µm for the upper filter, separated by polymeric pads and placed in a polymeric body having on its circumference levers and a locking element for a bayonet connection with the container. The filter system is installed in a container in a silicone pad by bayonet connection; for this purpose the filter-container connection point has sector slots.

The conical shape of the container for tissue treatment and dividing it into two chambers by a system of microfilters with 10 to 500 µm pore diameter provides an increase in efficiency, safety, and cost-effectiveness of the cell isolation method.

The device has isolated channels in the container side walls, wherein the inlet of each channel has recesses and sectoral slots for bayonet connection to the adapter fittings.

The device has a channel for biological material injection, terminating in a recess slot in the chamber for tissue treatment.

The device has a channel for reagent and wash buffer injection, terminating in a recess slot in the chamber for tissue treatment.

The device has a channel for extraction of supernatant containing cellular debris, residues of untreated stromal and adipose tissue, terminating at the filter attaching point in the tissue treatment chamber.

The device has a channel for end product extraction as stromal vascular fraction of adipose tissue, terminating at the container base in a cell-concentrating chamber.

The device can have additional channels for components injection or extraction terminating in a tissue treatment chamber and/or in a cell concentration chamber.

The device has a bacteriological filter for pressure equalization in the system.

The device has a lid, whereby the lid has locking elements in its circumference and connects to the container through a silicone pad by bayonet connection or thread.

The lid of the device can have holes for each of the channels and a bacteriological filter.

The lid can be connected to the container through a silicone pad by steel bolts; for this purpose the container has corresponding threaded holes.

The device has adapter fittings for each channel. Each adapter fitting has a back-flow valve. Each adapter fitting has a Luer-Lock type thread for connection to a syringe. Each adapter fitting has a screw cap. Each adapter fitting has silicon pads for tightness when connected to a container. Each adapter fitting has a locking element for connection to a container. Each adapter fitting is connected to a container through the lid and a silicone pad by bayonet connection.

The device may have a casing-cover, wherein the casing-cover is connected to the container via bayonet connection or thread.

The device can have blade-shaped legs as a container seat.

The device can have slots for fixing on the swinging bucket rotor. In this case, the device is supplied with internal steel armature along the slots.

The device inner surface is coated with an organosilicone and/or agarose film for surface hydrophobic and antiadhesive properties. To do this, the sterile and fat-free system is dried and the inner surfaces of the system and channels are treated with 0.1-10% polydimethylsiloxane solution in chloroform and/or 0.1-5% agarose solution. The treated system is placed for 1-5 hours in a loss-on-drying oven at temperature of 100°C to 300°C.

The device can have a 50-200 ml Luer-Lock syringe (not shown in the drawings) with an elongated nose, a screw cap and an enlarged piston handle for injection of biological material and extraction of the supernatant and the end product.

All device components, including container, lid, adapter fittings, adapter-fitting lids, filter housing, and system pads are made of polycarbonate and/or polypropylene and/or polyethylene and/or fluoroplastic. The pads and valves are made of silicone and/or rubber.

The method for extracting stromal vascular fraction of adipose tissue from lipoaspirate for the said invention comprises the following steps:
- lipoaspirate decantation and washing out from blood residues, wherein decantation is carried out within 10-30 minutes and the lipoaspirate wash is carried out with saline and/or Hank's balanced salt solution and/or Dulbecco modified Eagle medium at 37°C, wherein the lipoaspirate washing is carried out 1 to 5 times;
- lipoaspirate fermentative treatment with an enzyme mixture of collagenase I and/or collagenase II and/or thermolysin and/or dispase and/or trypsin, wherein the treatment is carried out at 37°C under a thermostat, with treatment by shaking at 10-200 rpm, wherein the treatment time is 30 to 60 minutes;
- separating the stromal vascular fraction from the cell debris and the stromal residues of the adipose tissue, wherein the separation is carried out with centrifugation at 500-2000 g for 15 to 45 minutes;
- deletion of cellular debris and adipose tissue stromal residues;
- end product washing out from enzyme residues, wherein the wash is carried out with saline and/or Hank's balanced salt solution and/or Dulbecco modified Eagle medium at 37°C, with the end product wash performed 1-5 times;
- concentration of the stromal vascular fraction, wherein the concentration is carried out with centrifugation at 500-2000 g for 15-45 minutes;
- wherein all process steps are carried out in the apparatus of the present invention;
- wherein the application of the biological material and its extraction from the device are carried out by using a syringe for injection and extraction of biological material.

The isolated stromal vascular fraction of adipose tissue can be used in biology and medicine as a cellular element for creation of tissue-engineering structures based on synthetic and natural materials.

The isolated stromal vascular fraction of adipose tissue can be used in biology and medicine as a cellular autologous graft for replacement and regeneration of hard and soft tissue defects, by injecting the stromal vascular fraction of adipose tissue into hard and/or soft tissues.

Thus, the biological tissue put in the system is washed out from the circulating blood residues; it is subjected to enzymatic treatment with a mixture of collagenases I and II, thermolysin, dispase, and trypsin, which causes stromal tissue lysis and outflow of stromal vascular fraction into the medium. At the next stage, the cellular component and stromal and adipose tissue residues are separated by centrifugation through a system of filters with different pore sizes, followed by washing out the cell fraction from the residual enzymes and its concentrating. As a result of the inner surface treatment with anti-adhesive media, no remains of circulating blood and cell fraction stick to the walls of the system, and they are completely washed out during the procedure. By using a mixture of enzymes, there is a complete lysis of stromal tissue and stromal vascular fraction outflow in the medium. Due to the presence of a system of filters with different pore diameters, optimal cell fraction isolation without admixtures of stromal and adipose tissue occurs. The presence of channels for introduction and extraction ending at different levels inside the system wall allows the total area of the system inner surface to be minimized, which reduces the risk of cell adhesion on the tube surface; allows the biological material and reagents to be introduced in a controlled near-wall manner, which reduces the physical effects on the biological material and increases the survival rate of the cells; allows the supernatant with residual adipose tissue stroma to be fully extracted without affecting the sediment; allows the sediment to be completely extracted in the form of a stromal vascular fraction of adipose tissue. The input valves on the channels ensure the process sterility inside the system. Clear plastic is used as the main material, which allows the visual control of the cell isolation process, introduction of biological material and reagents, extraction of the supernatant with residual stromal and adipose tissues, and extraction of the end stromal vascular fraction. The presence of graduation marks on the system surface allows for the visual control of the volume of the introduced biological material and reagents. The use of a syringe with a long tip allows the introduction and extraction of biological material and reagents without the risk of material contamination and liquid spillage outside the system.

The stromal vascular fraction of the adipose tissue isolated by using the claimed invention can be used for creation of tissue-engineered constructs based on synthetic and natural materials; for use as cellular autologous graft; as a method of treatment, repair or replacement of the tissue, including the injection of stromal vascular fraction of adipose tissue into hard and/or soft tissues.

### BRIEF DESCRIPTION OF THE DRAWINGS

Fig. 1 shows the device for isolating cell fractions from human and animal tissues, in exploded device view.
Fig. 2 shows the device for isolating cell fractions from human and animal tissues, in exploded sectional view.
Fig. 3 shows the device for isolating cell fractions from human and animal tissues, in assembled view.
Fig. 4 shows the device for isolating cell fractions from human and animal tissues, in assembled sectional view.
Fig. 5 shows the device for isolating cell fractions from human and animal tissues, in top view.
Fig. 6 shows a cell monolayer in culture p0 (passage No. 1), the 5th day. Phase contrast (magnification of x10).
Fig. 7 shows a cell monolayer in culture p1 (passage No. 1), the 5th day. Phase contrast (magnification of x10).

### EMBODIMENT OF THE INVENTION

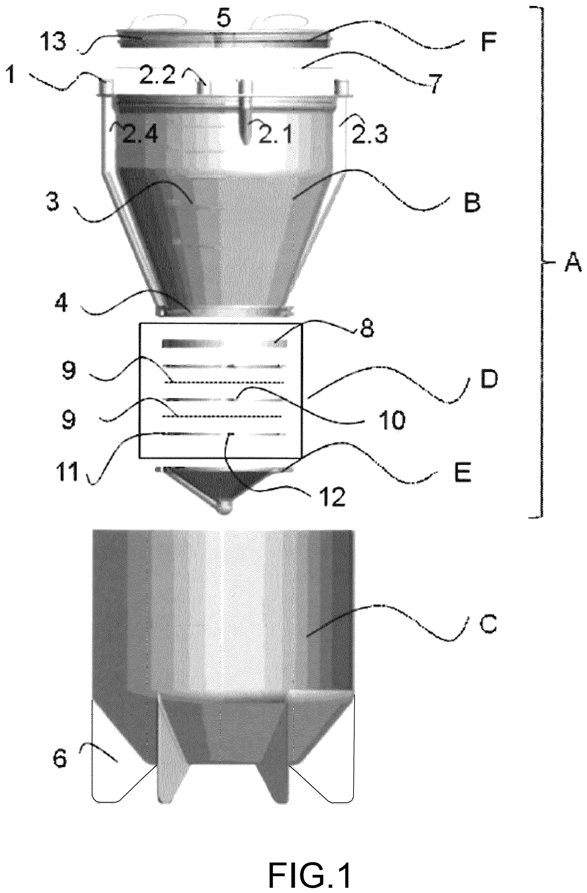

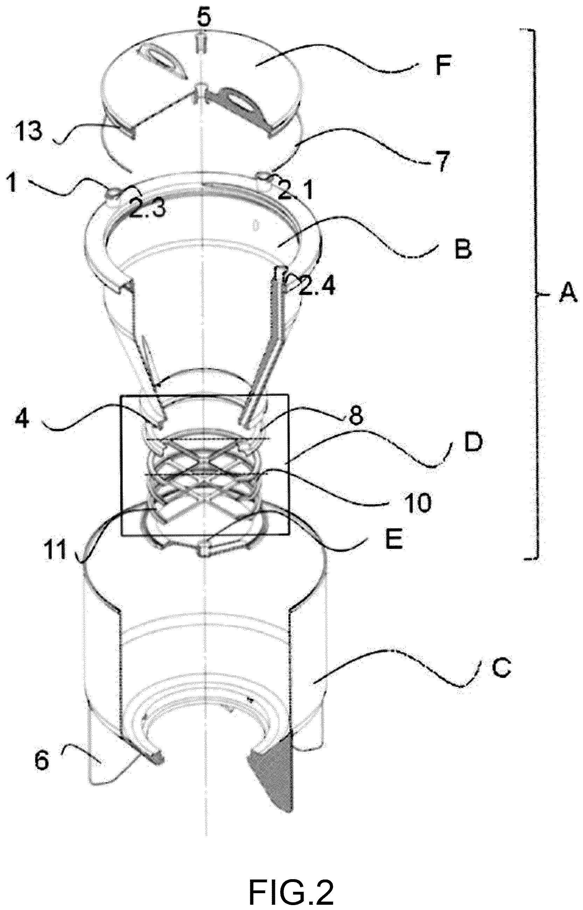

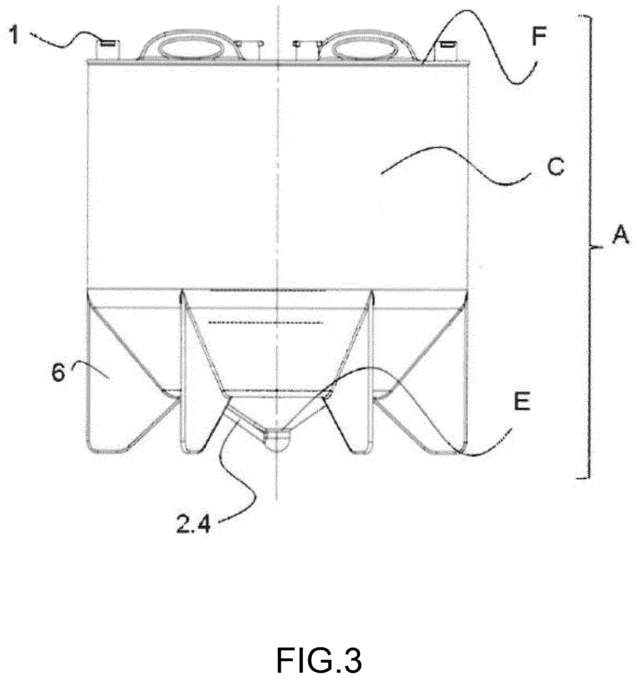

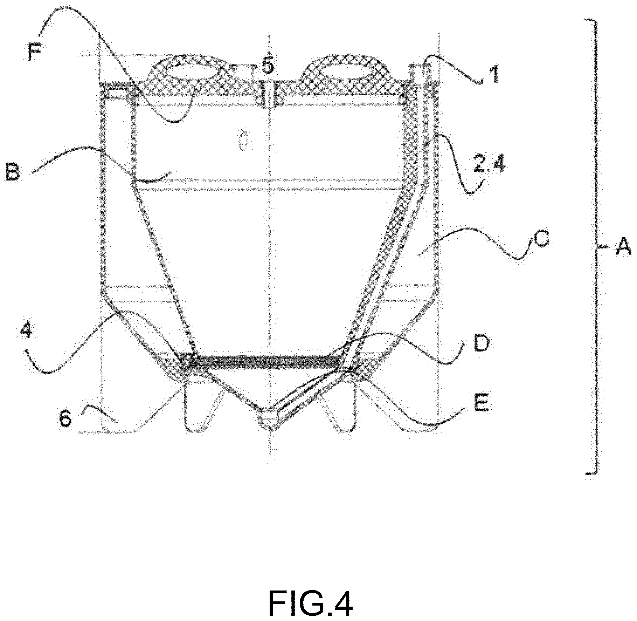

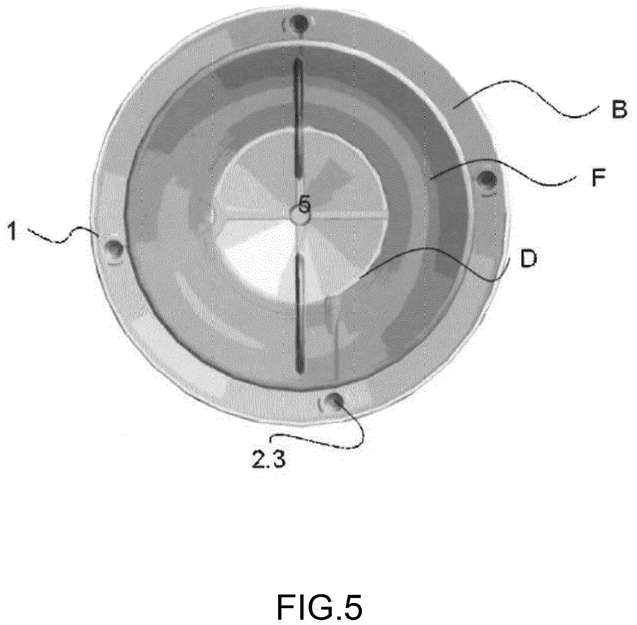

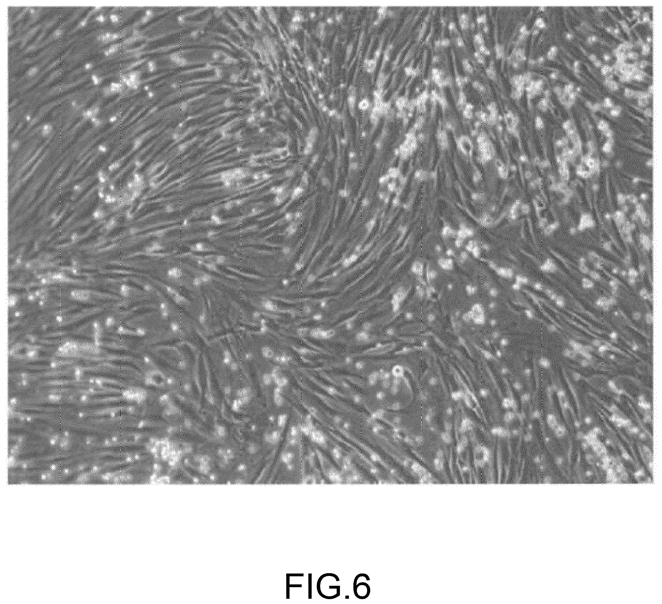

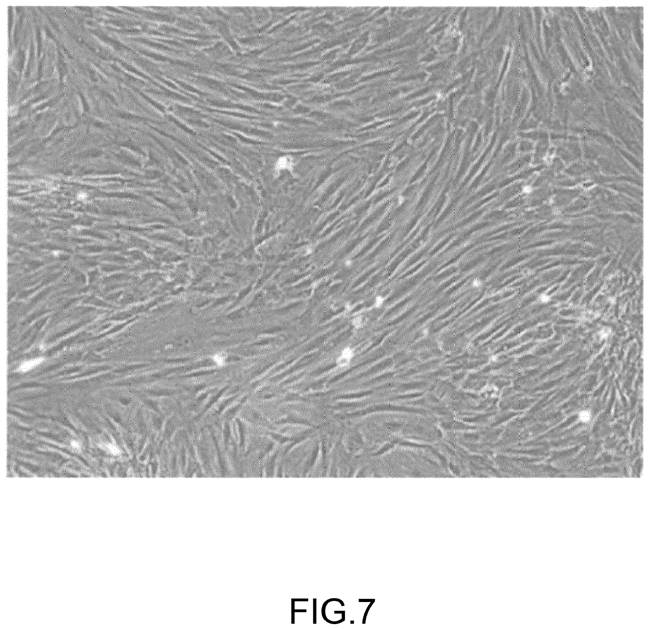

The gist of the disclosure can be explained by the drawings. Fig. 1 shows the device for isolating cell fractions from human and animal tissues, in exploded device view. Fig. 2 shows the device, in exploded sectional view. Fig. 3 shows the device, in assembled view. Fig. 4 shows the device, in assembled sectional view. Fig. 5 shows the device, in top view. Fig. 6 is a photomicrograph of the cell monolayer in culture p0 (passage No. 1), the 5th day. Phase contrast. Magnification of x10. Fig. 7 is a photomicrograph of the cell monolayer in culture p1 (passage No. 1), the 5th day. Phase contrast. Magnification of x10.

The device for isolating cell fractions from human and animal tissues is a closed fluid-tight system in the shape of a transparent conic vessel (A) with external graduation of the volume (3), set of channels (2.1, 2.2, 2.3, 2.4) and adapter fittings (1) with Luer-Lock connectors, fastened inside the transparent housing (C) by using bayonet joint (4); the vessel is separated with a system of microfilters (D) into two chambers - a chamber for tissue treatment (B) and a cell-concentrating chamber (E).

The microfilter system consists of at least two nylon microfilters (9) separated with polymer inserts (10) and placed in a polymer housing which consists of upper silicone seal (8) and base (11) with circumference levers and a locking element (12) for connections with the tissue treatment chamber (B) and the cell-concentrating chamber (E). The microfilter system is placed into the silicone seal within the vessel (8). The device has isolated channels (2.1, 2.2, 2.3, 2.4) in the vessel side walls; the inlet of each channel has recesses and sectoral slots (not indicated on the drawing) for bayonet joint to the adapter fittings. The device has a bacteriological filter (5) for pressure equalization in the system. The device has a lid (F) with at least one hole and with circumference locking elements; the lid is fastened to the vessel through a silicone seal (7) by bayonet connection or thread (13). The device has adapter fittings (1) for each channel. The device has a base/housing (C) with paddle-like feet (6).

### EMBODIMENT EXAMPLE

The given example is not intended to limit the invention but is offered solely as an illustration.

In general terms, the device operation represents a stepwise washing out and enzymatic treatment of adipose tissue in order to isolate the stromal vascular fraction. The lipoaspirate of adipose tissue is put into a fluid-tight vessel (A), namely in a tissue treatment chamber (B), through the channel (2.1) via a connection of the syringe with biological material to the adapter fitting (1). The biological tissue is washed out from the residual blood in a buffer solution injected through the channel (2.2). The excessive liquid is extracted through the channel (2.4). The biological tissue is treated with a mixture of proteolytic enzymes injected through the channel (2.2). The device is centrifuged and the stromal vascular fraction passes through the microfilter system (D) to the cell-concentrating chamber (E). The excessive liquid is extracted through the channel (2.3) and the sediment is washed out from the residual enzymes with buffer solution. The end stromal vascular fraction is extracted through the channel (2.4). The device operated with 3 samples of lipoaspirate derived from surgical liposuction. The lipoaspirate was extracted by using 50 mL Luer-Lock type syringes. 100 mL of the lipoaspirate were injected in the tissue treatment chamber (B) by washing down the sides of the system neatly through the channel (2.1) via the connection of the syringe (is not indicated on the drawing) to the adapter fitting (type 1), then the lid of the adapter fitting was closed tightly. 100 mL of Hank's balanced salt solution were injected through the channel (2.2) via the connection of the syringe (not indicated on the drawing) to the adapter fitting (type 1) and then the lid of adapter fitting was closed tightly. The system was put in a temperature-controlled (37°C) chamber with 100-150 RPM shaker for 15-20 minutes in order to wash out the biological material. The system was centrifuged at 500 g for 10 minutes. 100 mL of the sediment were thoroughly extracted through the channel (2.4) by using the syringe without overturning the system and then the lid was closed tightly. The washing-out cycle was repeated three times. 10 mL of a mixture of collagenase I, collagenase II, thermolysin, dispase, and trypsin were injected through the channel (2.2) via the connection of the syringe (not indicated on the drawing) to the adapter fitting (type 1), then the lid of the adapter fitting was closed tightly. 90 mL of Hank's balanced salt solution were injected through the channel (2.2) via the connection of the syringe (not indicated on the drawing) to the adapter fitting (type 1) and then the lid of the adapter fitting was closed tightly. The system was put in temperature-controlled (37°C) chamber with a 100-150 RPM shaker for 45 minutes in order to wash out the biological material. The system was centrifuged at 1000 g for 30 minutes. 190 mL of fat, stroma, debris, and residual erythrocytes were thoroughly extracted through the channel (2.3) via the connection of the syringe (not indicated on the drawing) to the adapter fitting (type 1) and then the lid of adapter fitting was closed tightly. 90 mL of Hank's balanced salt solution were introduced through the channel (2.2) via the connection of the syringe (not indicated on the drawing) to the adapter fitting (type 1) and then the lid of adapter fitting was closed tightly. The system was centrifuged at 1000 g for 5 minutes. The washing-out procedure was repeated three times. 190 mL of fat, stroma, and debris impurities were thoroughly extracted through the channel (2.3) by using the syringe without overturning the system, then the lid was closed tightly. 10 mL of autologous patient serum were injected through the channel (2.2). The system was put in a temperature-controlled (37°C) chamber with a 100-150 RPM shaker for 5 minutes in order to inactivate enzymes. The system was centrifuged at 1000 g for 5 minutes. 2 mL of stromal vascular fraction were extracted through the channel (2.4).

The obtained primary culture of stromal vascular fraction was characterized in terms of cell viability and expression of surface markers - CD90, CD105, CD73, CD34, CD11b, CD19, CD45, CD44 - immediately after the extraction, on the first and second passages.

No less than 100,000 events for each sample were analyzed. After treatment and centrifugation of the cell suspension according to the protocol, FSC and SSC, analyses were performed in order to determine the size of the study objects and the presence of intracellular inclusions.

The isolated fraction is characterized by "event purity" and low content of duplicates and debris. Optical characteristics were used for determining the lymphocytic and monocytic gate and target cell fraction. It was demonstrated that single cells composed 92% of 47,264 events (43,487 events in absolute terms), duplicates (aggregated cells) - 1.4%, debris (cellular debris from the sample) and erythrocytes - only 6.2%. The lymphocytic and monocytic gate composed 91.8% of the pooled single events, the target fraction - 7.5%. Meanwhile, the total concentration of stromal vascular fraction cells per unit volume was 66,000 cells per 100 µL.

In order to identify and analyze the regenerative fraction in an isolated sample, common markers of mature stem cells were used (CD90, CD73, CD105, CD44) .

The experimental results have shown that the isolated fraction contains up to 20% of CD90-positive cells, 0.24% of CD105-positive cells, and no less than 0.01% of CD44-positive cells.

The endoglin (CD 105) expression is typical for MSC after the passage; however, this marker can be expressed also on progenitor cells - mesenchymal angioblasts. Thus the percentage of regenerative cells in the isolated stromal vascular fraction is 0.5%.

Cell fraction viability analysis was performed, including apoptotic and dead cell calculation. The test was performed using FITC-labeled Annexin V which is affine to phosphatidylserine. The phosphatidylserine is localized on the surface of apoptotic cells and it forms one of the specific characteristic signals of apoptotic cells. The annexin test results showed that 1.02% of the study fraction cells are apoptotic cells, 0.04% are destructed cells, and 98.94% are live cells.

After the passage, a persistent increase on CD 90 and CD 105 expression was observed, which is typical for the mesenchymal stromal cell culture. On the 5th day of each passage, the cells achieved 100% confluence (monolayer), which is demonstrated on Fig. 3 and Fig. 4.

**Table 1. Results of staining the markers by using Human MSC Analysis Kit (BD, cat#562245) (CD90, CD105, CD73, CD34, CD11b, CD19, CD45, CD44)**

| **Marker** | **P0** | **P1** | **P2** |
|---|---|---|---|
| CD90 | - | ++ | ++ |
| CD73 | + | + | + |
| CD44 | + | - | - |
| CD105 | + | ++ | ++ |
| CD 34, 11b, 19, 45 | - | + | + |

| | | | |
|---|---|---|---|
| P0 - passage 0 (fig. 6). P1 - passage 1 (fig. 7). P2 - passage 2 (not indicated on the figures). | | | |

Hereinbefore, the device for isolating cell fractions from human and animal tissues was described. This device contains a fluid-tight vessel for tissue treatment with adapter fittings and a channel system. According to the invention, the vessel for tissue treatment is of conical shape and is separated into two chambers by a microfilter system with pore sizes of 10-500 microns, hermetically fastened with bayonet joint through silicone seal, where one chamber is used for tissue treatment and a second chamber is used for cell concentrating. The inner surface of the vessel has hydrophobic antiadhesive coating, and the area of the inner surface is minimized by using channels within the system wall, wherein one channel opens in the tissue treatment chamber, a second channel opens in the cell-concentrating chamber, and the pressure is equalized by using a bacteriological filter.

A method for isolating cell fractions from human and animal tissues by using the proposed device was also described hereinbefore. It includes decantation of a lipoaspirate; delicate washing out of the lipoaspirate in buffer solutions; enzymatic treatment by using a mixture of collagenases, thermolysin, dispase and trypsin at a temperature of 37ºC; centrifugation at 500-2000 g and filtration through microfilters with a pore size of 10-300 microns with the subsequent removal of fat and stromal tissue matter; repeated washing out, and concentration of the cell fraction.

Though the present invention has been described in detail with respect to exemplary embodiments that appear to be preferred, it is to be understood that these embodiments of the invention are for the purposes of illustration only. This description should not be considered as limiting the scope of the invention, since specialists in the field of medical biotechnology and cell technology, etc. may modify the stages of the described methods and devices in order to adapt them to specific devices or situations, but not beyond the scope of the appended claims. It is understood by the specialists in this field that different variations and modifications, including equivalent solutions, are possible within the scope of the invention as defined by the claims.

### REFERENCES

1. System for processing lipoaspirate cells EP 1921133 A2
2. Method and Apparatus for Separating a Material US 20110251041 A1
3. Regenerative cell extraction device WO 2013183797 A1
4. Device for separating adult stem cell US 20130344589 A1
5. System and methods for preparation of adipose-derived stem cells, US 20130012921
A1, WO 2014011213 A1

## Claims

1. A device for separating a stromal-vascular fraction from human or animal tissues, containing a fluid-tight vessel for tissue treatment having and a channel system within side walls of the vessel, wherein the inlet of each channel has recesses and sectoral slots for bayonet connection to the adapter fittings, said vessel for tissue treatment is of a conical shape and divided into two chambers by a microfilter system, the microfilter system contains at least two nylon microfilters, having pore diameters from 10 µm for the lower filter to 500 µm for the upper filter, and filters are separated by polymeric pads, the chambers are hermetically fastened with a bayonet connection through a silicone seal, wherein one chamber, namely a tissue-treatment chamber, is used for tissue treatment and the other chamber, namely a cell-concentrating chamber, is used to concentrate cells, wherein the inner surface of the vessel has a hydrophobic antiadhesive coating; one channel of the channel system opens into the tissue-treatment chamber, another channel of the channel system opens into the cell-concentrating chamber, and the vessel also contains a bacteriological filter configured to equalize pressure inside the device.

2. The device according to Claim 1, having paddle-like feet in the lower part of the cone.

3. The device according to Claim 1, wherein slots are present on the outside of the vessel for fastening the vessel into a swinging bucket rotor of a centrifuge, wherein the device is to be equipped with an internal steel armature along the slots.

4. The device according to Claim 1, having pumps for liquid feed and cell extraction.

5. A method for separating a stromal-vascular fraction from human or animal tissues by using the device according to any one of the claims 1-4, the method comprising: decanting a lipoaspirate; washing out delicately the lipoaspirate in buffer solutions; providing an enzymatic treatment of the lipoaspirate by using a mixture of collagenases, thermolysin, dispase, and trypsin at a temperature of 37 °C; performing centrifugation at 500-2000 g and filtering through microfilters with a pore size of 10-500 µm with the subsequent removal of fat and stromal tissue; repeating the washing out and concentration of the cell fraction.

6. A stromal vascular fraction of adipose tissue, obtained by using the device according to Claim 1 for creation of tissue-engineered constructs based on synthetic and natural materials.

7. The stromal vascular fraction of adipose tissue according to Claim 6 for use as cellular autologous graft.

## Patentansprüche

1. Vorrichtung zum Trennen einer stromal-vaskulären Fraktion von menschlichem oder tierischem Gewebe, die ein flüssigkeitsdichtes Gefäß zur Gewebebehandlung mit einem Kanalsystem innerhalb der Seitenwände des Gefäßes enthält, wobei der Einlass jedes Kanals Aussparungen und sektorale Schlitze für eine Bajonettverbindung mit Adapteranschlüssen aufweist,
wobei das Gefäß zur Gewebebehandlung eine konische Form aufweist und durch ein Mikrofiltersystem in zwei Kammern unterteilt ist, wobei das Mikrofiltersystem mindestens zwei Nylon-Mikrofilter mit Porendurchmessern von 10 µm für den unteren Filter bis 500 µm für den oberen Filter enthält und die Filter durch Polymerpads getrennt sind, wobei die Kammern mit einer Bajonettverbindung durch eine Silikondichtung hermetisch befestigt sind, wobei eine Kammer, genauer gesagt eine Gewebebehandlungskammer, zur Gewebebehandlung verwendet wird und die andere Kammer, genauer gesagt eine Zellkonzentrationskammer, zum Konzentrieren von Zellen verwendet wird, wobei die Innenfläche des Gefäßes eine hydrophobe Antihaftbeschichtung aufweist; wobei ein Kanal des Kanalsystems sich in die Gewebebehandlungskammer öffnet, ein anderer Kanal des Kanalsystems sich in die Zellkonzentrationskammer öffnet, und das Gefäß auch einen bakteriologischen Filter enthält, der dazu konfiguriert ist, den Druck innerhalb der Vorrichtung auszugleichen.

2. Vorrichtung nach Anspruch 1, die paddelartige Füße in dem unteren Teil des Kegels aufweist.

3. Vorrichtung nach Anspruch 1, wobei an der Außenseite des Gefäßes Schlitze zum Befestigen des Gefäßes in einem schwingenden Schaufelrotor einer Zentrifuge vorhanden sind, wobei die Vorrichtung mit einem inneren Stahlanker entlang der Schlitze ausgestattet sein soll.

4. Vorrichtung nach Anspruch 1, die Pumpen zur Flüssigkeitszufuhr und Zellextraktion aufweist.

5. Verfahren zum Trennen einer stromal-vaskulären Fraktion von menschlichem oder tierischem Gewebe unter Verwendung der Vorrichtung nach einem der Ansprüche 1 bis 4, wobei das Verfahren Folgendes umfasst: Dekantieren eines Lipoaspirats; vorsichtiges Auswaschen des Lipoaspirats in Pufferlösungen; Bereitstellen einer enzymatischen Behandlung des Lipoaspirats unter Verwendung einer Mischung aus Kollagenasen, Thermolysin, Dispase und Trypsin bei einer Temperatur von 37 °C; Durchführen einer Zentrifugation bei 500 bis 2000 g und Filtrieren durch Mikrofilter mit einer Porengröße von 10-500 µm mit anschließender Entfernung von Fett und Stromagewebe; Wiederholen des Auswaschens und der Konzentration der Zellfraktion.

6. Stromal-vaskuläre Fraktion von Fettgewebe, erhalten unter Verwendung der Vorrichtung nach Anspruch 1 zur Herstellung von Konstrukten aus Gewebezüchtungen auf der Basis von synthetischen und natürlichen Materialien.

7. Stromal-vaskuläre Fraktion von Fettgewebe nach Anspruch 6 zur Verwendung als zelluläres autologes Transplantat.

## Revendications

1. Dispositif pour séparer une fraction stroma-vasculaire à partir de tissus humains ou animaux, contenant un récipient étanche aux fluides pour le traitement des tissus et ayant un système de canaux à l'intérieur des parois latérales du récipient, dans lequel l'entrée de chaque canal présente des évidements et des fentes sectorielles pour un raccordement à baïonnette aux raccords adaptateurs,
ledit récipient pour le traitement des tissus est de forme conique et divisé en deux chambres par un système de microfiltre, le système de microfiltre contient au moins deux microfiltres en nylon, ayant des diamètres de pore allant de 10 µm pour le filtre inférieur à 500 µm pour le filtre supérieur, et les filtres sont séparés par des tampons polymères, les chambres sont fixées hermétiquement avec un raccordement à baïonnette par l'intermédiaire d'un joint en silicone, dans lequel une chambre, c'est-à-dire une chambre de traitement de tissu, est utilisée pour le traitement des tissus et l'autre chambre, c'est-à-dire une chambre de concentration de cellules, est utilisée pour la concentration des cellules, dans lequel la surface interne du récipient présente un revêtement antiadhésif hydrophobe, un canal du système de canaux s'ouvre dans la chambre de traitement de tissu, un autre canal du système de canaux s'ouvre dans la chambre de concentration de cellules, et le récipient contient également un filtre bactériologique configuré pour égaliser la pression à l'intérieur du dispositif.

2. Dispositif selon la revendication 1, ayant des pieds en forme de palette dans la partie inférieure du cône.

3. Dispositif selon la revendication 1, dans lequel des fentes sont présentes à l'extérieur du récipient pour la fixation du récipient dans un rotor à godets oscillants d'une centrifugeuse, dans lequel le dispositif doit être équipé d'une armature interne en acier le long des fentes.

4. Dispositif selon la revendication 1, ayant des pompes pour l'alimentation en liquide et l'extraction de cellules.

5. Procédé pour séparer une fraction stroma-vasculaire à partir de tissus humains ou animaux en utilisant le dispositif selon l'une quelconque des revendications 1 à 4, le procédé comprenant la décantation d'un produit de lipoaspiration ; le lavage délicat du produit de lipoaspiration dans des solutions tampons ; la fourniture d'un traitement enzymatique du produit de lipoaspiration en utilisant un mélange de collagénases, de thermolysine, de dispase et de trypsine à une température de 37 °C ; la mise en œuvre d'une centrifugation à 500 à 2000 g et d'une filtration à travers des microfiltres ayant une taille de pore de 10 à 500 µm avec l'élimination ultérieure de la graisse et du tissu stromal ; la répétition du lavage et la concentration de la fraction cellulaire.

6. Fraction stroma-vasculaire de tissu adipeux, obtenue en utilisant le dispositif selon la revendication 1 pour la création de constructions conçues à partir de tissus à base de matériaux synthétiques et naturels.

7. Fraction stroma-vasculaire de tissu adipeux selon la revendication 6 pour une utilisation comme greffe autologue cellulaire.
